# DEMANDE DE BREVET EUROPEEN

(11) **EP 4 773 337 A1**
(43) Date de publication de la demande: **08.07.2026**
(21) Numéro de dépôt: 25223259.0
(22) Date de dépôt: 15.12.2025
(51) Int. Cl.: H01M 10/48

(54) **COUVERTURE DE SÉCURITÉ POUR LA SURVEILLANCE DE BATTERIES ÉLECTROCHIMIQUES, À CAPTEURS DE TEMPÉRATURE**

(30) Priorité: 07.01.2025 FR 2500137
(71) Demandeur: Commissariat à l'Energie Atomique et aux Energies Alternatives, 75015 Paris (FR)
(72) Inventeur: DITTO, Aurélia, 38054 GRENOBLE CEDEX 09 (FR); BONNAUD, Céline, 38054 Grenoble cedex 09 (FR); WEICK, Clément, 38054 Grenoble cedex 09 (FR); DAUCHY, Julien, 38054 Grenoble cedex 09 (FR)
(74) Mandataire: INNOV-GROUP

(57) **Abrégé**

Couverture de sécurité pour la surveillance de batteries électrochimiques, comportant :
- une membrane souple (3), adaptée à entourer au moins partiellement au moins une batterie électrochimique (2) à surveiller en définissant une atmosphère confinée (8), cette membrane souple (3) présentant une face interne (5) destinée à être tournée vers la batterie électrochimique (2), et une face externe (6) destinée à être tournée vers l'extérieur ;
- au moins un capteur de température (4) disposé sur la face interne de la membrane souple (3) ;
- un module de contrôle (9) raccordé au capteur de température (4) et adapté à détecter un évènement d'augmentation de température de l'atmosphère confinée (8) à une vitesse supérieure à un seuil prédéterminé ;
- une interface d'alerte (10) adaptée à émettre un signal représentatif d'une alerte lorsque le module de contrôle (9) détecte ledit évènement.

## Description

### DOMAINE TECHNIQUE

L'invention concerne le domaine des batteries électrochimiques utilisées dans de nombreuses applications fixes ou mobiles, pour le stockage d'énergie électrique. Ces applications sont par exemple dans le domaine des véhicules électriques, des engins légers de mobilité, des accumulateurs stationnaires d'énergie électrique, du stockage tampon des solutions d'énergie renouvelable (panneaux photovoltaïques, éoliennes, etc.), ou tout autre moyen fixe ou mobile de stockage d'énergie électrique.

L'invention concerne plus particulièrement les moyens de surveillance, dans une optique de sécurité, pour ces batteries.

La forte progression des appareils, installations, et véhicules munis de batteries électrochimiques sur toutes les plages de puissance a nécessité des adaptations relatives aux questions de sécurité qui se posent lors de la présence de batteries dans des lieux très divers (ayant trait aux engins de mobilité et objets nomades) ainsi que de la présence de grandes quantités de batteries, et/ou de batteries de forte puissance dans les environnements des utilisateurs, dans les transports, ou dans les lieux de traitement de ces batteries.

Les batteries électrochimiques présentent en effet notamment des risques potentiels de fuite d'électrolyte corrosive et/ou inflammable, d'emballement thermique, de feu, d'explosion, etc.

Les batteries électrochimiques sont par exemple des batteries de chimie lithium ou nickel, des batteries au plomb, etc. ou tout autre type courant de batterie électrochimique. L'expression « batterie électrochimique » englobe ici les cellules élémentaires, les modules comportant plusieurs cellules, les batteries comportant plusieurs modules, les pack-batterie comportant plusieurs de ces batteries, ou tout autre sous-ensemble de batteries.

### ART ANTÉRIEUR

Les dispositifs actuellement connus pour la surveillance des batteries électrochimiques reposent en général sur les dispositifs BMS (« Battery Management System », en anglais) de la batterie, ou sur d'autres dispositifs électroniques connexes aux batteries ou aux groupes de batteries mis en œuvre.

Les dispositifs connus représentent un premier niveau de sécurité satisfaisant, lorsque les batteries sont équipées de tels dispositifs. Cependant, ces dispositifs risquent d'être défaillants lorsque la batterie est elle-même défaillante.

Pour certaines applications, pour certaines batteries non munies de ces dispositifs, et pour les situations où la constitution de la batterie est inconnue (phases de recyclage, de transport, de prévention, d'accident, etc.), les moyens connus ne fournissent pas un niveau de sécurité suffisant.

Les documents CN113571801 et EP4087032 décrivent une boite et un sac de stockage pour batterie.

### EXPOSÉ DE L'INVENTION

L'invention a pour but d'améliorer les moyens de sécurité pour la surveillance de batteries électrochimiques.

À cet effet, l'invention vise une couverture de sécurité conforme à la revendication 1.

Selon un autre objet, l'invention vise un procédé de surveillance d'une batterie électrochimique conforme à la revendication de procédé.

L'invention permet une surveillance des batteries électrochimiques avec un niveau de sécurité accru, de manière indépendante des batteries surveillées elles-mêmes, des conditions d'environnement de ces batteries, de l'identification de ces batteries, et de leur historique.

L'invention permet d'ajouter un niveau élevé de sécurité aux batteries qui sont dépourvues de moyens internes de diagnostic, aux batteries dont les moyens de surveillance sont défaillants, ou aux batteries dont la constitution n'est pas connue.

L'invention est particulièrement adaptée aux phases de vie qui sont en dehors des phases nominales de décharge, par exemple les phases de recyclage, lors de la prévention d'accidents, de la sécurisation des batteries impliquées dans un incident, la sécurisation de véhicules ou d'installations, le transport des batteries ou des véhicules, machines ou installations.

Cependant, l'invention peut être mise en œuvre dans toutes les phases d'utilisation de la batterie, notamment durant les phases de charge, qui peuvent nécessiter une surveillance.

Par exemple, les phases de vie relatives au recyclage des batteries vont être de plus en plus répandues et vont faire l'objet d'un nombre croissant de procédés, de stockage associé, et d'usines de recyclage appropriées dont le dimensionnent ira en augmentant. Ces phases de recyclage sont en effet règlementées, voire rendues obligatoires. Dans cet exemple, d'importantes quantités de batteries correspondant à de fortes puissances vont être transportées et stockées en vue des procédés de recyclage, et ces batteries peuvent être de constitution, de fonctionnement, et d'historique inconnus. L'invention permet de sécuriser ce type de stock de batteries aux propriétés incertaines, en détectant et en alertant sur un danger potentiel ou effectif.

Selon un autre exemple, les phases de transport des batteries sont des phases complexes nécessitant des identifications et des mesures spécifiques, ainsi que des agréments ou normalisations vis-à-vis des transporteurs. L'invention permet à tout transporteur d'ajouter un niveau de sécurité au service de transport, voire d'obtenir un agrément qui ne serait pas obtenu sans la couverture de sécurité, et ce pour des batteries même de nature et d'historique inconnus.

Selon un autre exemple, en cas d'incident (accident, incendie, inondation, etc.) impliquant une installation ou un véhicule comportant des batteries, les dommages potentiels aux batteries peuvent déclencher des évènements indésirables dans un second temps, tels que emballement thermique et départ de feu, projection d'électrolyte, dégazage, etc. L'invention permet de sécuriser une zone d'accident, en mettant les batteries sous surveillance, afin de protéger les intervenants de secours, et ne pas créer de facteur aggravant.

Selon un autre exemple, lors du transport d'une installation endommagée ou d'un véhicule endommagé ou accidenté, les phases de transport de ces éléments potentiellement dangereux peuvent être sécurisées par l'invention.

Selon un autre exemple, les phases de charge d'une batterie peuvent être des séquences présentant un risque, pour des raisons de perturbation du milieu environnant, d'éventuels défauts électriques, de défaillance des chargeurs, etc. L'invention permet également de sécuriser de manière dynamique ces phases de charge.

L'invention permet de plus de compléter la surveillance des batteries par une alerte sur l'ensemble des aléas pouvant survenir à une batterie, tels que : chocs mécaniques, décharge profonde, surcharge, court-circuit externe, température ambiante trop élevée.

L'invention présente une solution maniable et facile à mettre en place, sans nécessiter de formation technique.

L'invention permet également une grande modularité dans la mise en œuvre des alertes qui peuvent être sonores, visuelles, à distance, transmises sur un réseau, etc.

La couverture de sécurité et le procédé selon l'invention peuvent comporter les caractéristiques additionnelles contenues dans les revendications dépendantes, seules ou en combinaison.

### PRÉSENTATION DES FIGURES

D'autres caractéristiques et avantages de l'invention ressortiront de la description non limitative qui suit, en référence aux dessins annexés dans lesquels :
- la figure 1 illustre schématiquement en perspective une batterie prévue pour être mise sous surveillance ;
- la figure 2 illustre une couverture de sécurité mise en place sur la batterie ;
- la figure 3 illustre schématiquement la couverture de sécurité mise à plat, du côté de sa face interne ;
- la figure 4 illustre schématiquement la couverture de sécurité mise à plat, du côté de sa face externe ;
- la figure 5 est une vue schématique en coupe de la couverture de sécurité mise en place sur la batterie ;
- la figure 6 est un diagramme illustrant le procédé de surveillance de batteries ;
- la figure 7 est un diagramme illustrant des variantes du procédé.

Les éléments similaires et communs aux divers modes de réalisation portent les mêmes numéros de renvoi aux figures.

### DESCRIPTION DÉTAILLÉE

L'invention concerne une couverture de sécurité 1 pour la surveillance de batteries électrochimiques. Cette couverture de sécurité 1 est destinée à recouvrir, directement ou indirectement, au moins une partie d'une batterie 2, ou d'un groupe de batteries, que l'on souhaite mettre sous surveillance.

La couverture de sécurité 1 est destinée à la mise sous surveillance, de manière exceptionnelle ou systématique, d'une batterie électrochimique pour surveiller notamment tout emballement thermique, départ de feu, dégazage, etc.

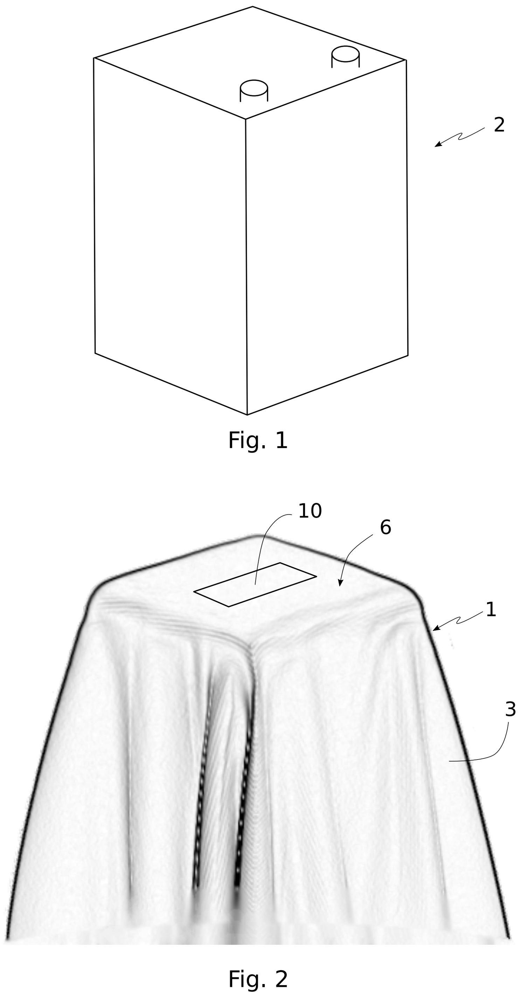

La couverture de sécurité 1 est mise en place directement sur la batterie 2 ou sur un sous-ensemble contenant la batterie 2, voire un véhicule contenant la batterie 2. La figure 1 illustre un exemple d'une batterie 2 électrochimique que l'on souhaite mettre sous surveillance.

Par exemple, la batterie 2 peut-être une batterie lithium-ion mise sous surveillance en vue de son stockage ou de son transport avant recyclage, ou en vue de sécuriser un équipement ou un véhicule accidenté sur la scène de l'accident, ou lors du transport des éléments accidentés.

La couverture de sécurité 1 comporte une membrane souple 3 qui est adaptée à entourer au moins partiellement la batterie 2. La figure 2 illustre un exemple dans lequel la batterie 2 de la figure 1 est posée sur une surface, et la couverture de sécurité 1 est disposée de manière à recouvrir complètement la batterie 2.

La membrane souple 3 est prévue pour une fonction de recouvrement et est réalisée dans un matériau souple adéquat tel qu'un textile technique, tissé ou non tissé.

De préférence, le matériau de la membrane souple 3 est résistant aux hautes températures, voire résistant au feu. Plus précisément, la membrane 3 est de préférence réalisée dans un matériau souple dont la température de fusion est supérieure à une température prédéterminée. Par exemple, cette température prédéterminée peut être d'environ 800 °C lorsque la membrane souple 3 est réalisée dans un tissu de fibres de verre dont le point de fusion se situe aux alentours des 800 °C.

La membrane souple 3 présente les caractéristiques principales suivantes :
- elle est adaptée à créer une atmosphère confinée 8 autour de la batterie 2. L'étanchéité absolue aux gaz n'est pas nécessaire, **il** suffit que la membrane souple 3 produise un relatif confinement, même avec une étanchéité relative comme celle fournie par un textile tissé ;
- elle présente une résistance mécanique suffisante pour le support des capteurs 4, 7 décrits plus loin ;
- elle présente une souplesse suffisante pour recouvrir la batterie. La couverture de sécurité 1 peut avantageusement recouvrir différents types et formes de batterie, dans une certaine plage de gabarit, afin d'être adaptable.

La membrane souple 3 peut être réalisée dans tout textile technique présentant ces caractéristiques. Il peut s'agir avantageusement d'un aérogel de silice, d'un tissu de fibres de verre, d'un tissu de silice, qui sont ignifugés et résistant à la perforation. Ces tissus peuvent avantageusement être enduits d'un polymère ou d'un minéral, par exemple de polyuréthane ou de vermiculite.

La membrane souple 3 comporte une face interne 5 destinée à être tournée vers la batterie 2 lorsque la couverture de sécurité 1 est en place sur cette dernière, et une face externe 6 tournée vers l'extérieur et visible ou accessible par l'utilisateur.

La couverture de sécurité 1 peut comporter sur sa face interne 5 :
- au moins un capteur de température 4, voire un maillage d'une pluralité de capteurs de température 4 ; et/ou
- au moins un capteur d'oxyde de carbone 7, voire un maillage d'une pluralité de capteurs d'oxyde de carbone 7.

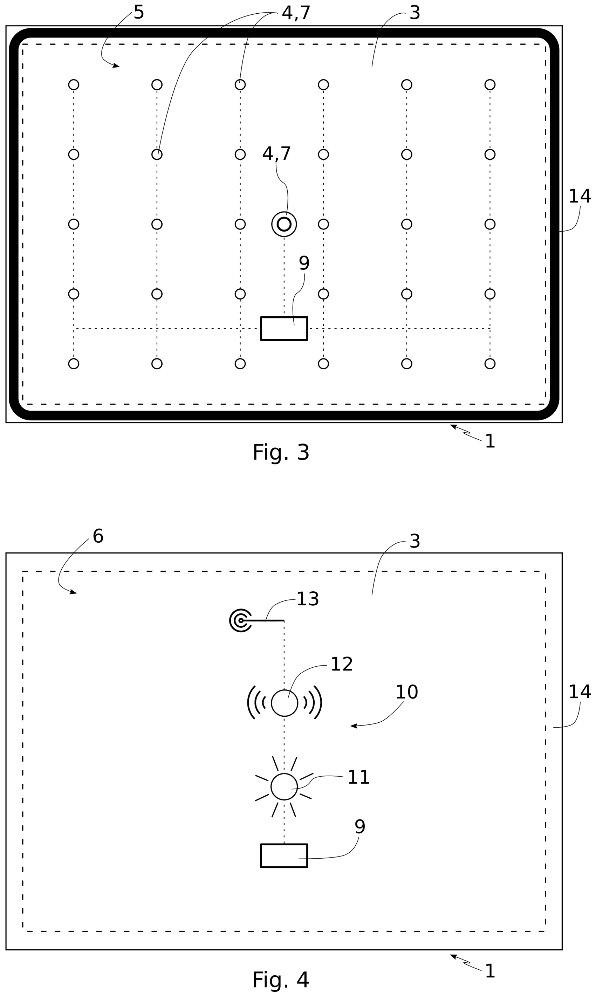

La figure 3 est une vue de dessus d'une couverture de sécurité 1 déployée et mise à plat, la face interne 5 étant visible. Le maillage de capteurs de température 4 et/ou de capteurs d'oxyde de carbone 7 est schématiquement représenté, ainsi que ses liaisons électriques en pointillés. Ces liaisons peuvent être sous forme de câbles, ou d'autres moyens de communication.

Dans le présent exemple, le maillage de capteurs 4, 7 est constitué d'une matrice surfacique de capteurs 4, 7, ainsi disposée à la surface de la face interne 5 de la membrane souple 3.

La figure 3 illustre le cas où la couverture de sécurité 1 comporte un maillage de capteurs 4, 7 qui est avantageusement centré sur la face interne 5.

La figure 3 illustre également le cas de figure où la couverture de sécurité 1 comporte un unique capteur 4, 7, ce dernier étant avantageusement situé en position centrale sur la face interne 5.

Ces deux cas de figure peuvent être combinés.

Le maillage de capteurs 4, 7 est destiné à quadriller au moins une portion de l'atmosphère confinée 8 (l'atmosphère confinée par la membrane souple 3). Plus ce maillage est fin, plus la surveillance est efficace mais plus elle nécessite de capteurs 4, 7.

Le maillage de capteurs 4, 7 est défini comme comportant au moins un capteur 4, 7 (adapté par exemple au cas d'une couverture de sécurité 1 de petite dimension pour la surveillance d'une petite batterie 2). Le maillage comporte un nombre de capteurs 4, 7 adapté aux dimensions maximales de batterie pour lesquelles la couverture de sécurité 1 est prévue.

Avantageusement, le maillage de capteurs 4, 7 présente une densité surfacique de capteurs sensiblement de 1 capteur par 10 cm² de membrane souple 3, sur au moins une portion de la face interne 5 de la membrane souple 3.

Dans l'exemple illustratif de la figure 3, la couverture de sécurité 1 comporte un maillage de trente capteurs 4, 7 sur la face interne 5 de la membrane souple 3. Le maillage de capteurs 4, 7 est ici illustré sous forme d'une matrice de capteurs 4, 7 alignés en lignes et colonnes. En variante, les capteurs 4, 7 peuvent être répartis selon toute autre dessin géométrique approprié, voire aléatoirement répartis, pour une forme particulière de batterie, par exemple en spirale, en cercles concentriques, etc.

Chacun des capteurs 4, 7 du maillage est fixé sur la membrane souple 3 par tous moyens connus tels que : couture, collage, sertissage, etc. Les capteurs 4, 7 peuvent également être intégrés au tissu formant la membrane souple 3, par intégration de microcapteurs dans le tissage.

Le maillage de capteurs 4, 7, dans sa disposition surfacique, permet de suivre l'évolution localisée de la température ou du taux d'oxyde de carbone dans l'atmosphère confinée 8, en vue du traitement exposé plus loin.

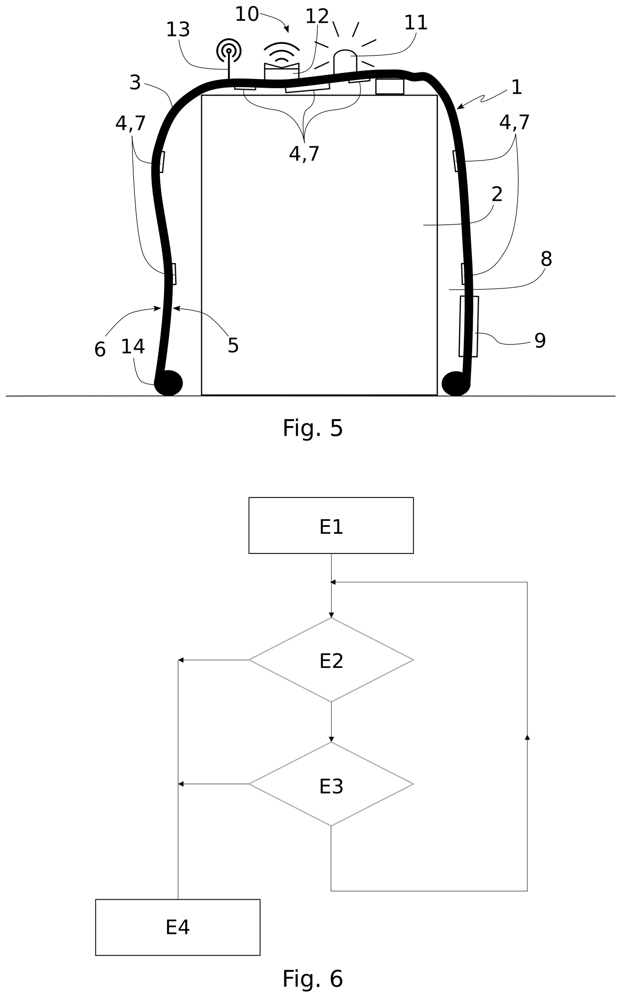

La figure 5 est une vue schématique en coupe d'une batterie 2 avec une couverture de sécurité 1 en place. La figure 5 illustre une répartition des capteurs 4, 7 autour d'une portion de la batterie 2.

Le ou les capteurs 4, 7 peuvent être les seuls capteurs présents dans la couverture, ou être associés à des capteurs complémentaires.

Comme illustré à la figure 3, la couverture de sécurité 1 peut comporter au moins un capteur d'oxyde de carbone 7 disposé sur la face interne 5 de la membrane souple 3. Dans un exemple, la couverture de sécurité 1 comporte un seul capteur d'oxyde de carbone 7 qui permet de mesurer l'évolution de la teneur d'un gaz de la famille des oxydes de carbone, présent dans l'air de l'atmosphère confinée 8.

La couverture de sécurité 1 peut comporter autant de capteurs d'oxyde de carbone 7 que nécessaire, étant entendu qu'un seul capteur d'oxyde de carbone 7 est suffisant pour de nombreuses dimensions de batterie, les gaz se diffusant rapidement dans le volume de l'atmosphère confinée 8. Dans le cas d'un capteur d'oxyde de carbone 7 unique, la position centrale est préférée.

Dans un mode de réalisation préféré, le capteur d'oxyde de carbone est un capteur de dioxyde de carbone et de monoxyde de carbone, qui est donc adapté à mesurer le taux de dioxyde de carbone et de monoxyde de carbone dans l'air de l'atmosphère confinée 8.

Le ou les capteurs d'oxyde de carbone 7 peuvent être réalisés par toute technologie connue permettant cette détection, et peuvent être fixés sur la membrane souple 3 de toute manière connue, comme les capteurs de température 4, de sorte que la partie active du capteur se trouve sur la face interne 5 de la membrane souple 3.

Le ou les capteurs d'oxyde de carbone peuvent être les seuls capteurs présents dans la couverture, ou être associés à des capteurs complémentaires. Dans le mode de réalisation présenté, ils sont cependant associés à des capteurs de température.

La couverture de sécurité 1 comporte de plus un module de contrôle 9 qui est raccordé aux différents capteurs 4, 7. Le module de contrôle 9 peut être un dispositif électronique classique, par exemple un microcontrôleur et ses périphériques, permettant de recueillir et traiter le signal des capteurs 4, 7.

Le module de contrôle 9 peut être adapté à détecter un évènement relatif à la température de l'atmosphère confinée 8 (et donc de la batterie), localisé ou diffus, lorsque la vitesse d'augmentation de cette température est supérieure à un seuil prédéterminé. Le maillage de capteurs de température 4 permet ainsi de quadriller l'atmosphère confinée 8 (qui est confinée par la membrane souple 3) de sorte qu'un point chaud au niveau de la batterie 2 puisse être détecté, grâce au maillage de capteurs de température 4 qui fourni un maillage de l'évolution de la température autour du point chaud. Le module de contrôle 9 est donc adapté à recueillir l'information de température instantanée des capteurs de température 4, à détecter et déterminer la vitesse d'augmentation de la température, et à la comparer au seuil prédéterminé programmé.

Par exemple, ce seuil prédéterminé peut être fixé à 1 °C par seconde. Ainsi, le module de contrôle 9 détecte un évènement de température lorsqu'il identifie une augmentation de température, sur au moins l'un des capteurs de température 4, supérieure à 1 °C par seconde.

Même lorsque la couverture de sécurité n'est équipée que pour mesurer la température, grâce à un ou plusieurs capteurs de température 4, elle permet de détecter et alerter de façon rapide lors un échauffement anormal et/ou un départ de feu.

Le module de contrôle 9 peut également être adapté à détecter un évènement relatif aux gaz d'oxyde de carbone dans l'atmosphère confinée 8, lorsque la vitesse d'augmentation du taux d'un gaz d'oxyde de carbone est supérieure à un seuil prédéterminé. Le module de contrôle 9 est donc adapté à recueillir l'information de taux instantané du ou des capteurs de gaz oxyde de carbone 7, à détecter et déterminer la vitesse d'augmentation de ce taux, et à la comparer au seuil prédéterminé programmé.

Par exemple, ce seuil prédéterminé peut être fixé à 25 ppm par seconde pour la vitesse d'augmentation du taux de dioxyde de carbone, et à 5 ppm par seconde pour la vitesse d'augmentation du taux de monoxyde de carbone. Ainsi, le module de contrôle 9 détecte un évènement de gaz d'oxyde de carbone lorsqu'il identifie une augmentation du taux respectif de ces gaz supérieure à 25 ppm par seconde ou à 5 ppm par seconde.

Même lorsque la couverture de sécurité n'est équipée que pour mesurer le taux d'oxyde de carbone, grâce à un ou plusieurs capteurs oxyde de carbone 7 dans l'atmosphère confinée 8, elle permet de détecter et alerter lors d'une modification de ce taux. Ceci est typiquement représentatif d'un évènement dans l'atmosphère confinée pouvant être lié à d'un éventage (autrement appelé dégazage) d'une cellule de la batterie 2. Dans le cas d'une batterie de grande taille, ou intégré au sein d'une système massif, cette détection liée à la diffusion de gaz peut avoir lieu avant une détection d'une élévation significative de la température du système, qui est thermalisé par la quantité de matériau présent.

La couverture de sécurité 1 comporte de plus une interface d'alerte 10 qui est prévue pour déclencher un signal représentatif d'une alerte lorsque le module de contrôle 9 détecte au moins l'un des évènements décrits.

L'interface d'alerte 10 est prévue pour alerter un utilisateur ou un dispositif externe lorsque le module de contrôle 9 détecte l'un desdits évènements. L'interface d'alerte 10 peut être constituée par tout dispositif électronique ou microélectronique classique, et comporte au moins un moyen d'alerte. Par exemple, l'interface d'alerte 10 peut comporter un dispositif d'alerte visuelle 11, un dispositif d'alerte sonore 12, et/ou un dispositif d'alerte déportée 13.

Le dispositif d'alerte visuelle 11 peut être, par exemple, une ou plusieurs LED indiquant le déclenchement d'une alerte. Ce dispositif d'alerte visuelle 11 peut également donner une information plus complète par un afficheur, un code couleur, ou un code de clignotement, pour donner des détails sur l'alerte en question, comme exposé plus loin.

Le dispositif d'alerte sonore 12 peut être tout moyen connu capable d'émettre un avertissement sonore, une alarme, etc. Ce dispositif d'alerte sonore 12 peut également donner une information plus complète par l'émission d'un code auditif, ou en langage naturel, pour donner des détails sur l'alerte en question, comme exposé plus loin.

Le dispositif d'alerte déportée 13 peut être tout dispositif connu adapté à une communication déportée, par exemple un dispositif de radiocommunication, sur une fréquence adaptée (par exemple wifi, bluetooth, 4G, etc.) ou un dispositif filaire connecté à des moyens d'alerte déportés par rapport à la couverture de sécurité, et permettant notamment la connexion à un réseau informatique et à l'internet, pour une information délivrée sur des terminaux fixes ou mobiles.

L'interface d'alerte 10 peut également comporter tout autre dispositif d'alerte adéquat permettant d'émettre une alerte particulière vers un utilisateur ou un système.

Lorsque le module de contrôle 9 détecte l'un des évènements précités, l'interface d'alerte 10 est activée et émet un signal d'alerte indiquant que la batterie 2 a été détectée comme présentant un danger :
- un emballement thermique localisé de la batterie (détecté grâce à une forte augmentation de température sur certains capteurs de température 4 seulement) ;
- un emballement thermique généralisé de la batterie (détecté grâce à une forte augmentation de température sur l'ensemble du maillage de capteurs de température 4) ;
- un feu au sein de la batterie (détecté grâce à une augmentation de température à la fois très importante et très localisée, et une augmentation rapide du taux de gaz d'oxyde de carbone).

La couverture de sécurité 1 peut de plus comporter des capteurs additionnels, qui permettent soit d'ajouter des alertes supplémentaires, soit de donner des informations sur l'historique de la batterie. Dans les exemples décrits, les capteurs additionnels peuvent être :
- des capteurs d'intensité lumineuse, pour la détection d'arcs électriques produits pas la batterie ;
- des accéléromètres, ou autres capteurs de vibration, pour la détection des chocs subis par la batterie ;
- des capteurs de composés organiques volatils (COV), pour la détection des fuites d'électrolyte ;
- des capteurs de température ambiante de la batterie, disposés sur la face externe 6 de la membrane souple 3, pour la détection d'une situation d'exposition de la batterie à une température ambiante risquant de la dégrader.

Les alertes correspondantes peuvent être émises par tout moyen choisi dans les possibilités de l'interface d'alerte, avec des signaux visuels, auditifs, ou des informations transmises par radiocommunication.

Les capteurs d'intensité lumineuse sont disposés sur la face interne 5 de la membrane souple 3 et reliés au module de contrôle 9 de sorte que le module de contrôle 9 puisse détecter une vitesse d'augmentation de l'intensité lumineuse au-delà d'un seuil prédéterminé (par exemple, un seuil de 200 lux par seconde). Un tel capteur d'intensité lumineuse est adapté à détecter un arc électrique se produisant au niveau de la batterie 2.

De préférence, de tels capteurs d'intensité lumineuse sont également disposés sous forme d'un maillage de capteurs d'intensité lumineuse, par exemple suivant une densité de 1 capteur par 50 cm² de surface de la membrane souple 3.

De préférence, la membrane souple 3 est également opaque de sorte que l'atmosphère confinée 8 reste dans le noir et n'est pas perturbée par la lumière externe à la couverture de sécurité 1.

Les accéléromètres sont disposés sur la membrane souple 3, au plus proche de la batterie, et reliés au module de contrôle 9 de sorte que le module de contrôle 9 puisse détecter les chocs d'une intensité supérieure à un seuil prédéterminé, et à compter ces évènements de chocs (par exemple, un seuil de 8 g d'accélération). De tels accéléromètres sont adaptés à détecter les chocs entrainant un endommagement de la batterie, susceptible de représenter un danger.

La mesure peut être réalisée par un seul accéléromètre.

Le ou les capteurs de température ambiante sont disposés sur la face externe 6 de la membrane souple 3 et sont reliés au module de contrôle 9 de sorte que le module de contrôle 9 puisse détecter une température supérieure à un seuil critique. Les batteries lithium-ion, par exemple, sont sensibles aux températures élevées. Le seuil d'alerte peut être placé par exemple à 50 °C.

La couverture de sécurité 1 comporte par ailleurs de préférence des moyens pour garantir le recouvrement adéquat de la batterie 2, et pour garantir le maintien en place de la membrane souple 3. La couverture de sécurité 1 peut avantageusement comporter à cet effet un rebord périphérique lesté 14. Par exemple, la membrane souple 3 peut comporter un ourlet sur son pourtour, et cet ourlet est rempli d'un matériau de lest souple ou pulvérisé, comme illustré à la figure 3. Ce rebord périphérique lesté 14 permet d'assurer le recouvrement de la batterie 2 comme illustré sur la vue en coupe de la figure 5.

La figure 6 illustre le procédé selon l'invention, qui permet de mettre sous surveillance la batterie 2 grâce à la couverture de sécurité 1.

La batterie 2 est tout d'abord repérée et sa mise sous surveillance décidée. Il s'agit par exemple de batteries à l'historique inconnu (stock en attente de recyclage), de batteries liées à un accident, de batteries que l'on souhaite transporter, etc.

Dans une étape E1 (figure 6), la couverture de sécurité 1 est mise en place sur la batterie 2 de sorte que la membrane souple 3 recouvre au moins partiellement la batterie 2.

À l'étape E2 est réalisée une évaluation du gaz oxyde de carbone dans l'air de l'atmosphère confinée 8. Par exemple, la vitesse d'augmentation du taux de CO2 et du taux de CO dans cet air peut être mesuré et comparé à sa valeur seuil qui, dans cet exemple illustratif, peut être respectivement de 25 ppm par seconde et de 5 ppm par seconde. Si l'un de ces deux gaz oxyde de carbone est détecté avec une telle vitesse d'augmentation, le module de contrôle 9 passe à l'étape E4 dans laquelle l'interface d'alerte 10 est activée pour exprimer une alerte relative à un départ de feu.

À l'étape E3 est réalisée une évaluation de la température dans l'atmosphère confinée 8, sur le contour de la batterie 2. Par exemple, la vitesse d'augmentation de la température autour de la batterie 2 peut être mesurée et comparée à sa valeur seuil qui, dans cet exemple illustratif, peut être de 1 °C par seconde. Si une telle vitesse d'augmentation de la température est détectée, le module de contrôle 9 passe à l'étape E4 dans laquelle l'interface d'alerte 10 est activée pour exprimer une alerte relative à un ou plusieurs points chauds sur la batterie, ou un emballement thermique.

Le module de contrôle 9 reboucle sur les deux étapes E2 et E3 tant qu'une alerte E4 n'est pas donnée. L'ordre de détection E2 et E3 n'a pas d'importance. La détection de température peut être faite avant la détection de gaz. Le procédé peut comporter une seule des étapes E2 ou E3.

L'alerte au niveau de l'étape E4 peut être donnée de manière sélective, pour donner plus d'informations sur l'évènement, en donnant des précisions sur l'évènement détecté grâce aux possibilités de l'interface d'alerte 10. L'interface d'alerte 10 est ainsi adaptée à émettre un signal représentatif d'une alerte indiquant un niveau d'alerte, c'est-à-dire donnant une information supplémentaire sur la gravité ou le type d'alerte.

L'alerte donnée par l'interface d'alerte 10 peut ainsi présenter des informations différentes, plus précises, en émettant des signaux lumineux ou des signaux sonores différents pour chaque type d'alerte, lorsque l'interface d'alerte comporte des dispositifs d'alerte visuel 11 ou sonore 12, ou en donnant plus d'informations, par exemple sur un écran ou sur un réseau, grâce au dispositif d'alerte radio 13 s'il est présent.

Une alerte visuelle par signaux lumineux associée à une alerte sonore, par exemple, est particulièrement efficace dans un espace de stockage où de nombreuses batteries sont mises sous surveillance, l'utilisateur identifiant immédiatement, dans un premier temps, qu'une alerte est activée au niveau du stock (par le signal auditif), pour identifier dans un second temps quelle est la batterie concernée (par le signal visuel). Une consultation d'un écran relatif à la couverture de sécurité 1 de la batterie concernée pourra donner finalement un diagnostic plus précis de l'évènement.

De même, l'interface d'alerte 10 peut émettre des signaux relatifs à la localisation du point chaud sur une batterie de grandes dimensions.

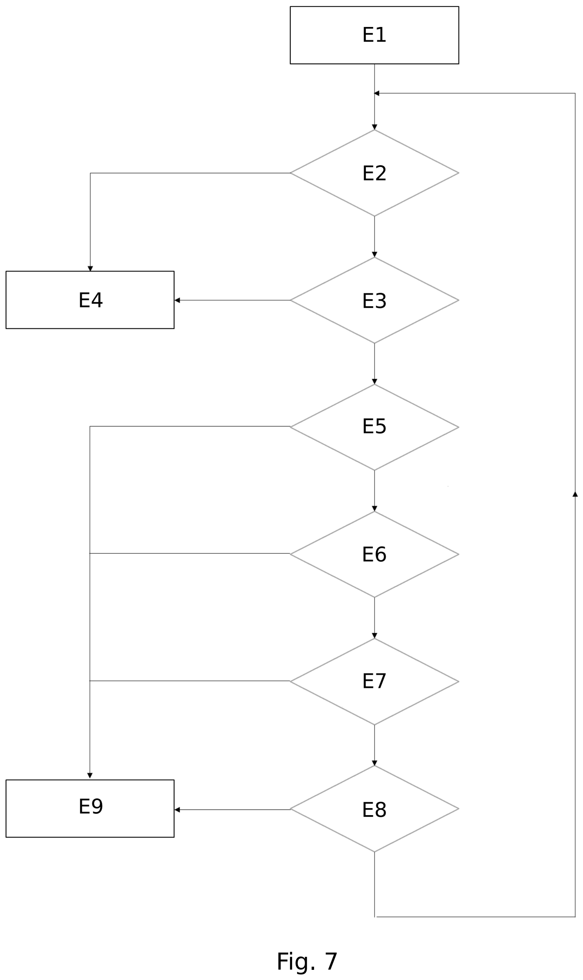

La figure 7 illustre un autre mode de réalisation du procédé, utilisant les capteurs additionnels décrits précédemment. Ainsi, en plus des étapes E1 à E4 décrites précédemment, le procédé comporte ici :
- une étape E5 dans laquelle est réalisée une évaluation de l'intensité lumineuse dans l'atmosphère confinée 8, grâce aux capteurs d'intensité lumineuse. Par exemple, la vitesse d'augmentation de l'intensité lumineuse peut être mesurée et comparée à sa valeur seuil qui, dans cet exemple illustratif, peut être de 200 lux par seconde. Si une telle vitesse d'augmentation d'intensité lumineuse est détectée, le module de contrôle 9 passe à l'étape E9 dans laquelle l'interface d'alerte 10 est activée pour exprimer une alerte relative à un arc électrique produit par la batterie 2 ;
- une étape E6 dans laquelle est réalisée une évaluation des chocs subis par la batterie 2, grâce aux accéléromètres. Par exemple, l'intensité des chocs subis peut être mesurée par la couverture de sécurité 1 et comparée à sa valeur seuil qui, dans cet exemple illustratif, peut être de 8 g. Si un tel choc est détecté, le module de contrôle 9 passe à l'étape E9 dans laquelle l'interface d'alerte 10 est activée pour exprimer un avertissement indiquant que la batterie 2 a subit un choc susceptible de l'endommager, et qu'elle peut être à ce titre défaillante ;
- une étape E7 dans laquelle est réalisée une évaluation de la présence de COV dans l'atmosphère confinée 8, grâce aux capteurs de COV. Par exemple, la présence de COV peut être mesurée et comparée à sa valeur seuil qui, dans cet exemple illustratif, peut être une valeur plancher en fonction des capteurs qui détecte la simple présence de COV dans l'atmosphère 8. Si une telle présence de COV est détectée, le module de contrôle 9 passe à l'étape E9 dans laquelle l'interface d'alerte 10 est activée pour exprimer une alerte relative à une fuite d'électrolyte dans la batterie 2 ;
- une étape E8 dans laquelle est réalisée une évaluation de la température subie par la batterie 2, grâce aux capteurs de température ambiante. Par exemple, la température ambiante, hors de l'atmosphère confinée, peut être mesurée et comparée à sa valeur seuil qui, dans cet exemple illustratif, peut être de 50 °C. Si une telle température est détectée, le module de contrôle 9 passe à l'étape E9 dans laquelle l'interface d'alerte 10 est activée pour exprimer un avertissement indiquant que la batterie 2 a subit une température trop élevée, susceptible de l'endommager.

Par ailleurs, le dispositif d'alerte 10 peut de plus comporter un module de coupure adapté à déconnecter un équipement de charge de la batterie. Ce module de coupure est destiné à être relié par exemple à un chargeur de la batterie, ou à l'alimentation d'un tel chargeur, pour une batterie 2 que l'on souhaite mettre en surveillance pendant sa charge. Il peut par exemple s'agir d'une prise gigogne comportant un relai piloté et à intercaler entre une prise électrique murale et le câble d'alimentation du chargeur de batterie. Ainsi, selon une variante du procédé, en cas de détection d'évènements, en plus d'activer les alertes, l'interface d'alerte 10 peut couper le courant de charge pour stopper ou ralentir l'incident en cours. Le déclenchement d'un système externe de sécurité, par exemple un système d'extinction ou d'inertage du local, peut également être effectué au travers d'une interface sur le dispositif d'alerte, par exemple au travers d'un relai à contact sec qui est mis en conduction lors d'une alerte.

L'activation de l'interface d'alerte 10 peut conduire à l'émission d'un signal qui est non seulement représentatif d'une alerte indiquant la détection d'un desdits évènements, mais qui est de plus représentatif de la localisation de la zone de capteurs dans laquelle a été détectée ledit évènement. L'interface d'alerte 10 est alors adaptée à émettre un signal représentatif de la localisation de l'évènement, par exemple pour localiser la batterie concernée lorsque la couverture de sécurité 1 recouvre plusieurs batteries, ou pour localiser la zone concernée sur une batterie. Pour la mise en œuvre de cette caractéristique, la couverture de sécurité comporte de préférence plusieurs capteurs, voire un maillage de capteurs.

D'autres variantes de réalisation peuvent être mises en œuvre. Par exemple, la couverture de sécurité peut comporter une membrane souple faite de toute autre matière adéquate, tissée ou non tissée (polymère déformable, etc.), présentant une souplesse suffisante pour le recouvrement d'une batterie 2.

De même, tout type de batterie, de sous-ensemble comportant des batteries, voire de véhicules munis de ces batteries, devant être mis sous surveillance peut bénéficier de la couverture de sécurité 1 qui est alors adaptée avec des dimensions adéquates.

La couverture de sécurité peut également comporter tout autre capteur connu pouvant ajouter des fonctions supplémentaires, raccordé au module de contrôle 9 et à l'interface d'alerte 10.

## Revendications

1. Couverture de sécurité pour la surveillance de batteries électrochimiques, **caractérisée en ce qu'**elle comporte :
- une membrane souple (3), adaptée à entourer au moins partiellement au moins une batterie électrochimique (2) à surveiller en définissant une atmosphère confinée (8), cette membrane souple (3) présentant une face interne (5) destinée à être tournée vers la batterie électrochimique (2), et une face externe (6) destinée à être tournée vers l'extérieur ;
- au moins un capteur de température (4) disposé sur la face interne de la membrane souple (3) ;
- un module de contrôle (9) raccordé au capteur de température (4) et adapté à détecter un évènement d'augmentation de température de l'atmosphère confinée (8) à une vitesse supérieure à un seuil prédéterminé ;
- une interface d'alerte (10) adaptée à émettre un signal représentatif d'une alerte lorsque le module de contrôle (9) détecte ledit évènement.

2. Couverture de sécurité selon la revendication 1, **caractérisée en ce que** ledit au moins un capteur de température comporte un maillage de capteurs de température (4) disposés sur la face interne de la membrane souple (3) et raccordés au module de contrôle (9).

3. Couverture de sécurité selon la revendication 2, **caractérisée en ce que** le maillage de capteurs de température (4) est constitué d'une matrice surfacique de capteurs de température (4), disposée à la surface de la face interne (5) de la membrane souple (3).

4. Couverture de sécurité selon l'une des revendications 2 ou 3, **caractérisée en ce que** le maillage de capteurs de température (4) présente une densité surfacique de capteurs sensiblement de 1 capteur par 10 cm² sur au moins une portion de la face interne (5) de la membrane souple (3).

5. Couverture de sécurité selon l'une des revendications précédentes, **caractérisée en ce que** l'interface d'alerte (10) est adaptée à émettre un signal représentatif d'une alerte indiquant la localisation de la zone de capteurs dans laquelle a été détecté ledit évènement.

6. Couverture de sécurité selon l'une des revendications précédentes, **caractérisée en ce qu'**elle comporte en outre au moins un capteur d'oxyde de carbone (7) disposé sur la face interne (5) de la membrane souple (3), et **en ce que** le module de contrôle (9) est raccordé audit au moins un capteur d'oxyde de carbone (7) et est adapté à détecter un évènement d'augmentation du taux d'un gaz oxyde de carbone dans l'atmosphère confinée (8) à une vitesse supérieure à un seuil prédéterminé, l'interface d'alerte (10) étant adaptée à émettre un signal représentatif d'une alerte lorsque le module de contrôle (9) détecte ledit évènement.

7. Couverture de sécurité selon l'une des revendications précédentes, **caractérisée en ce que** la membrane souple (3) comporte un rebord périphérique lesté (14).

8. Couverture de sécurité selon l'une des revendications précédentes, **caractérisée en ce que** l'interface d'alerte (10) est adaptée à émettre un signal différencié en fonction de l'évènement détecté par le module de contrôle (9).

9. Couverture de sécurité selon l'une des revendications précédentes, **caractérisée en ce qu'**elle comporte de plus un maillage de capteurs d'intensité lumineuse disposé sur la face interne (5) de la membrane souple (3), et **en ce que** le module de contrôle (9) est adapté à détecter de plus un évènement d'augmentation d'intensité lumineuse à une vitesse supérieure à un seuil prédéterminé.

10. Couverture de sécurité selon l'une des revendications précédentes, **caractérisée en ce que** la membrane souple (3) est opaque.

11. Couverture de sécurité selon l'une des revendications précédentes, **caractérisée en ce qu'**elle comporte de plus au moins un accéléromètre disposé sur la face interne (5) de la membrane souple (3), et **en ce que** le module de contrôle (9) est adapté à détecter de plus un évènement de choc subi à une accélération supérieure à un seuil prédéterminé.

12. Couverture de sécurité selon l'une des revendications précédentes, **caractérisée en ce qu'**elle comporte de plus au moins un capteur de composés organiques volatils disposé sur la face interne (5) de la membrane souple (3), et **en ce que** le module de contrôle (9) est adapté à détecter de plus un évènement de présence d'un composé carbonaté.

13. Couverture de sécurité selon l'une des revendications précédentes, **caractérisée en ce qu'**elle comporte en outre au moins un capteur de température ambiante disposé sur la face externe (6) de la membrane souple (3), en contact avec l'air ambiant, et **en ce que** le module de contrôle (9) est adapté à détecter de plus un évènement d'augmentation de la température ambiante au-delà d'un seuil prédéterminé.

14. Couverture de sécurité selon l'une des revendications précédentes, **caractérisée en ce que** l'interface d'alerte (10) comporte de plus un module de coupure adapté à déconnecter un équipement de charge de la batterie et/ou à déclencher un système d'inertage de la batterie, et/ou à déclencher un système d'extinction de la batterie.

15. Procédé de surveillance d'une batterie électrochimique, avec une couverture de sécurité selon l'une des revendications 1 à 14, **caractérisé en ce qu'**il comporte les étapes suivantes :
- mettre en position la couverture de sécurité (1) sur la batterie (2) ;
- détecter grâce au module de contrôle (9) un évènement d'augmentation de température de l'atmosphère confinée (8) à une vitesse supérieure à un seuil prédéterminé ;
- activer l'interface d'alerte (10).
